# EUROPEAN PATENT SPECIFICATION

(11) **EP 2 813 257 B1**
(45) Date of publication and mention of the grant of the patent: **04.05.2016**
(21) Application number: 13171427.1
(22) Date of filing: 11.06.2013
(51) Int. Cl.: A61M 39/10, A61M 39/24, A61M 39/26

(54) **Leak proof needleless medical connector**
Leckfreier nadelloser medizinischer Konnektor
Connecteur médical sans aiguille étanche

(43) Date of publication of application: 17.12.2014
(73) Proprietor: Tsai, Hsi-Chin, Taipei Hsien (TW)
(72) Inventor: Tsai, Hsi-Chin, Taipei Hsien (TW)
(74) Representative: Dossmann, Gérard

(56) References cited:
- WO-A1-2006/062912
- WO-A1-2010/127461
- WO-A2-2011/064738
- US-A1- 2012 059 334
- US-A1- 2012 277 688

## Description

The present invention relates generally to medical injectors, and more particularly to a needleless medical connector for preventing leakage of medication.

A medical injector is used to inject medication into a patient. A conventional medical injector comprises a syringe fitted with a needle. Medication is injected from the syringe into a patient through the needle. However, the needle is unsafe and medical personnel may accidentally get stabbed by the needle. Accordingly, a needleless medical injector without using a needle has been developed.

When the needleless medical injector is in use, a needleless medical connector is screwed into the needleless medical injector and a dosing unit is then screwed into the needleless medical connector. Thus, a flow channel is formed within the needleless medical connector and the dosing unit to allow passage of medication and injection of the medication into a patient. After the necessary medication has been dispensed, the dosing unit is removed from the needleless medical connector. However, current needleless medical connectors have poor sealing property such that leakage of the medication may occur when the dosing unit is removed. Document US 2012/277688 A1 describes a leak proof needleless medical connector according to the preamble of claim 1.

To overcome the shortcoming, the present invention provides a leak proof needleless medical connector that can prevent leakage of medication.

A leak proof needleless medical connector in accordance with the present invention comprises a housing, an actuator, a resilient member and an adapter. The housing includes a valve tube. The valve tube has a bottom annular blocking projection extending from an inner surface of the valve tube. The actuator is movably disposed within the housing. The resilient member is disposed within the housing and includes in sequence a lower compression tube, a column and an upper compression tube. The lower compression tube and the upper compression tube are resiliently compressible and expansible. The column has at least one through hole defined therethrough. An interior of the lower compression tube communicates with an interior of the upper compression tube through the at least one through hole of the column. A bottom valve stem is disposed in the lower compression tube. The lower compression tube is mounted around the valve tube of the housing and the bottom valve stem is mounted in the valve tube. A gap is formed between the bottom valve stem and the valve tube. The gap does not communicate with the interior of the upper compression tube. A bottom end of the bottom valve stem abuts the bottom annular blocking projection of the valve tube to close an open bottom of the valve tube. A top valve stem is disposed in the upper compression tube. The adapter is mounted on a top of the housing such that the resilient member is mounted between the adapter and the actuator. An interior of the adapter does not communicate with the at least one through hole of the column because of the said top valve stem. In use, a dosing unit is screwed into the needleless medical connector. Thus, a flow channel is formed within the needleless medical connector and the dosing unit to allow passage of medication.
FIG. 1 is a perspective view of a first embodiment of the leak proof needleless medical connector in accordance with the present invention;
FIG. 2 is an exploded perspective view of the leak proof needleless medical connector in FIG. 1;
FIG. 3 is an exploded cross-sectional perspective view of the leak proof needleless medical connector in FIG. 1;
FIG. 4 is a cross-sectional side view of the leak proof needleless medical connector in FIG. 1;
FIG. 5 is a cross-sectional side view of the leak proof needleless medical connector in FIG. 1 shown in an actuated state;
FIG. 6 is an operational perspective view of the leak proof needleless medical connector in FIG. 1 showing that the needleless medical connector is engaged with a needleless medical injector;
FIG. 7 is an operational perspective view of the leak proof needleless medical connector in FIG. 1 with the needleless medical injector, showing that the needleless medical connector is engaged with one of the dosing units;
FIGS. 8 to 10 are operational cross-sectional side views of the leak proof needleless medical connector in FIG. 1 with the needleless medical injector and the dosing unit;
FIG. 11 is an exploded perspective view of a second embodiment of the leak proof needleless medical connector in accordance with the present invention;
FIG. 12 is an exploded cross-sectional perspective view of the leak proof needleless medical connector in FIG. 11;
FIG. 13 is a cross-sectional side view of the leak proof needleless medical connector in FIG. 11;
FIG. 14 is a cross-sectional side view of the leak proof needleless medical connector in FIG. 11 shown in an actuated state;
FIGS. 15 to 17 are operational cross-sectional side views of the leak proof needleless medical connector in FIG. 11 with a needleless medical injector and a dosing unit;
FIG. 18 is an exploded perspective view of a third embodiment of the leak proof needleless medical connector in accordance with the present invention;
FIG. 19 is an exploded cross-sectional perspective view of the leak proof needleless medical connector in FIG. 18;
FIG. 20 is a cross-sectional side view of the leak proof needleless medical connector in FIG. 18;
FIG. 21 is a cross-sectional side view of the leak proof needleless medical connector in FIG. 18 shown in an actuated state;
FIG. 22 is an exploded perspective view of a fourth embodiment of the leak proof needleless medical connector in accordance with the present invention;
FIG. 23 is an exploded cross-sectional perspective view of the leak proof needleless medical connector in FIG. 22;
FIG. 24 is a cross-sectional side view of the leak proof needleless medical connector in FIG. 22;
FIG. 25 is a cross-sectional side view of the leak proof needleless medical connector in FIG. 22 shown in an actuated state; and
FIGS. 26 to 29 are enlarged perspective views of spherical compression tubes of the leak proof needleless medical connectors in accordance with the present invention.

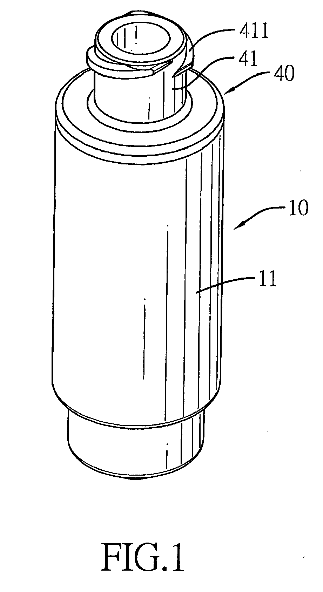

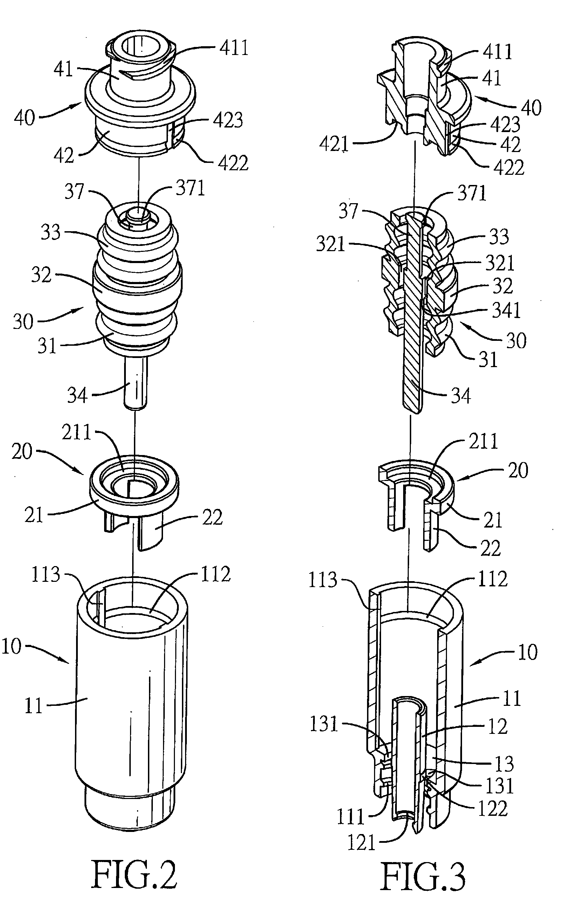

With reference to FIGS. 1 to 4, a leak proof needleless medical connector in accordance with the present invention comprises a housing 10, an actuator 20, a resilient member 30 and an adapter 40.

The housing 10 includes an outer casing 11, a valve tube 12 and an annular portion 13. A bottom portion of the outer casing 11 is provided with internal threads 111. The valve tube 12 is disposed within the outer casing 11 and adjacent to the internal threads 111, and the valve tube 12 has an inner surface, an outer surface, a bottom annular blocking projection 121 and an abutting ring 122. The bottom annular blocking projection 121 extends from a bottom end of the inner surface of the valve tube 12. The abutting ring 122 extends from the outer surface of the valve tube 12. The annular portion 13 is formed between the outer casing 11 and the valve tube 12 and has a plurality of longitudinal guide holes 131 defined therethrough.

The actuator 20 is hollow, is movably disposed within the outer casing 11 of the housing 10 and includes a collar 21 and a plurality of guide members 22. The collar 21 is mounted around the valve tube 12 of the housing 10 above the annular portion 13 and has a top surface, a bottom surface and a first annular receiving groove 211. The first annular receiving groove 211 is formed in the top surface of the collar 21. The guide members 22 extend downward from the bottom surface of the collar 21. The guide members 22 correspond to and are inserted respectively through the guide holes 131 of the annular portion 13 of the housing 10 to allow the actuator 20 to move longitudinally only within the housing 10. The abutting ring 122 of the valve tube 12 of the housing 10 abuts inner surfaces of the guide members 22.

The resilient member 30 is disposed within the outer casing 11 of the housing 10 above the actuator 20 and includes in sequence a lower compression tube 31, a column 32 and an upper compression tube 33. The lower compression tube 31 and the upper compression tube 33 are resiliently compressible and expansible. The column 32 has at least one longitudinal through hole 321 defined therethrough. An interior of the lower compression tube 31 communicates with an interior of the upper compression tube 33 through the at least one through hole 321 of the column 32. The lower compression tube 31 is mounted around the valve tube 12 of the housing 10 and a bottom end of the lower compression tube 31 is received in the first annular receiving groove 211 of the collar 21 of the actuator 20. A space 35 is formed between the valve tube 12 and the lower compression tube 31. A top end of an inner surface of the lower compression tube 31 abuts a top end of the outer surface of the valve tube 12 such that the space 35 does not communicate with the at least one through hole 321 of the column 32. A bottom valve stem 34 is disposed in the lower compression tube 31 and is mounted in the valve tube 12. A gap 36 is formed between the bottom valve stem 34 and the valve tube12. The gap 36 does not communicate with the interior of the upper compression tube 33. A bottom end of the bottom valve stem 34 abuts the bottom annular blocking projection 121 of the valve tube 12 to close an open bottom of the valve tube 12. Atop valve stem 37 is disposed in the upper compression tube 33.

The adapter 40 is hollow, is mounted on a top of the outer casing 11 of the housing 10 and includes a top annular member 41 and a bottom annular member 42. The top annular member 41 is provided with external threads 411. The bottom annular member 42 is inserted into the top of the outer casing 11 of the housing 10 such that a bottom end of the resilient member 30 abuts the collar 21 of the actuator 20 and a top end of the resilient member 30 abuts the bottom annular member 42 of the adapter 40. An interior of the adapter 40 does not communicate with the at least one through hole 321 of the column 32 because of the said top valve stem 37.

FIGS. 1 to 5 show a first embodiment of the leak proof needleless medical connector of the present invention.

The outer casing 11 of the housing 10 has an inner surface, an annular locking projection 112 and a plurality of spaced longitudinal locking ribs 113. The annular locking projection 112 and the longitudinal locking ribs 113 extend from the inner surface of the outer casing 11.

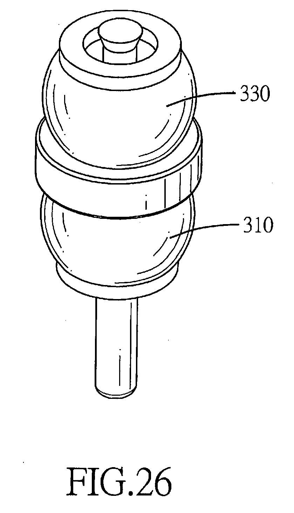

The bottom valve stem 34 extends downward from a central portion of a bottom surface of the column 32. The top valve stem 37 extends upward from a central portion of a top surface of the column 32. The column 32 has a plurality of through holes 321 defined therethrough. The through holes 321 are spaced around the bottom valve stem 34 and the top valve stem 37. The bottom valve stem 34 has a flange 341 at its top end. The flange 341 is inserted into a top of the gap 36 such that the gap 36 does not communicate with the through holes 321 of the column 32. The top valve stem 37 has an enlarged portion 371 at its top end. The enlarged portion 371 abuts a bottom end of an inner surface of the bottom annular member 42 of the adapter 40 such that the interior of the upper compression tube 33 does not communicate with the interior of the adapter 40. Walls of the lower and upper compression tubes 31, 33 may be waved, or, with reference to FIG. 26, walls of the lower and upper compression tubes 310, 330 may be spherical.

The bottom annular member 42 of the adapter 40 has a bottom surface, an outer surface, a second annular receiving groove 421, an annular locking groove 422 and a plurality of spaced longitudinal locking channels 423. The second annular receiving groove 421 is formed in the bottom surface of the bottom annular member 42. The top end of the upper compression tube 33 is received in the second annular receiving groove 421. The annular locking groove 422 and the longitudinal locking channels 423 are formed in the outer surface of the bottom annular member 42. The annular locking projection 112 of the outer casing 11 of the housing 10 corresponds to and engages the annular locking groove 422 to prevent the adapter 40 from being removed from the housing 10. The longitudinal locking ribs 113 of the outer casing 11 of the housing 10 correspond to and engage the longitudinal locking channels 423 to prevent the adapter 40 from rotating relative to the housing 10.

In the first embodiment, the leak proof needleless medical connector comprises four components including the housing 10, the actuator 20, the resilient member 30 and the adapter 40. The lower compression tube 31, the column 32, the upper compression tube 33, the bottom valve stem 34 and the top valve stem 37 are integrally formed to construct the resilient member 30.

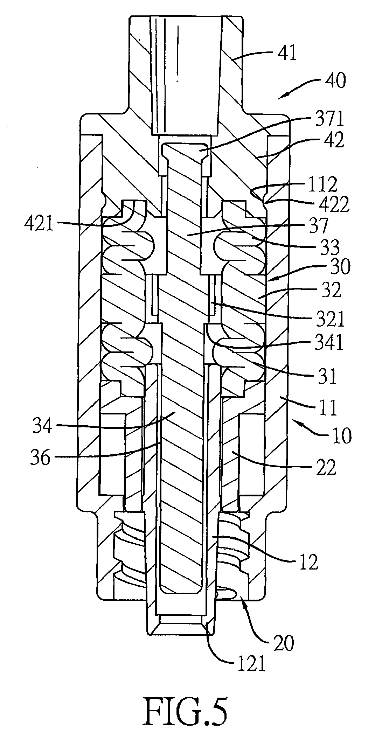

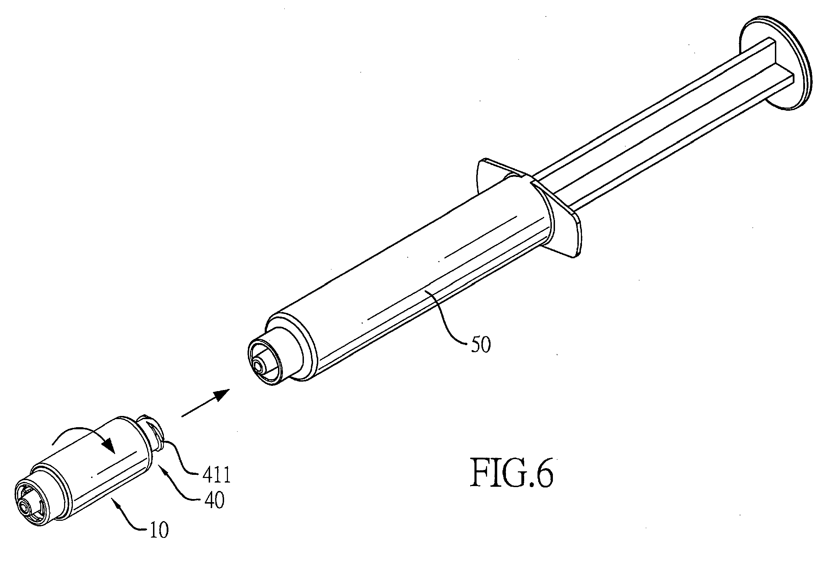

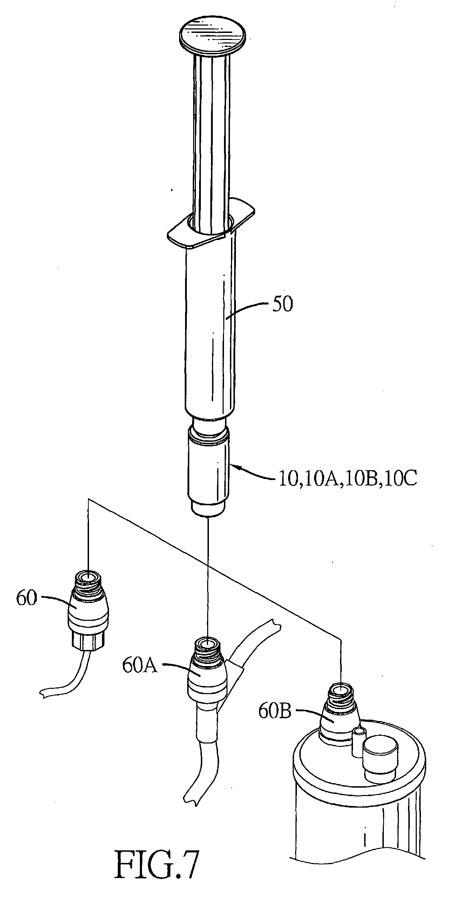

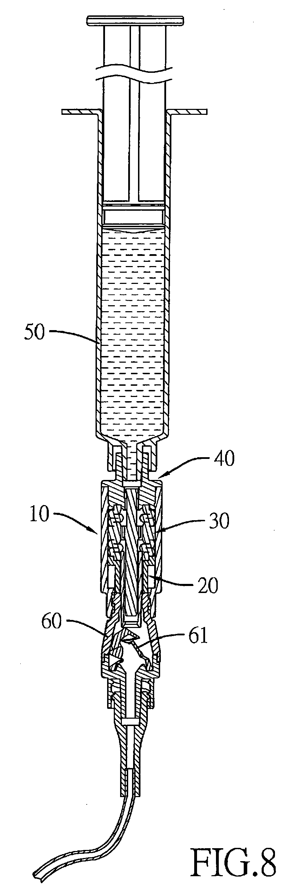

With reference to FIG. 6, when the first embodiment of the leak proof needleless medical connector is in use, the external threads 411 of the top annular member 41 of the adapter 40 are screwed into internal threads of a needleless medical injector 50. With reference to FIG. 7, external threads of an appropriate dosing unit 60, 60A, 60B are then screwed with the internal threads 111 of the outer casing 11 of the housing 10. The following description describes a situation in which the dosing unit 60 is used. With reference to FIGS. 5 and 8, the dosing unit 60 is screwed into the housing 10 to move the guide members 22 of the actuator 20 longitudinally within the housing 10, such that the actuator 20 is moved up to compress the lower and upper compression tubes 31, 33. Under this circumstance, the bottom end of the bottom valve stem 34 is moved away from the bottom annular blocking projection 121 of the valve tube 12 of the housing 10 to open the open bottom of the valve tube 12, the flange 341 of the bottom valve stem 34 is moved away from the top of the gap 36 to allow the gap 36 to communicate with the through holes 321 of the column 32, and the enlarged portion 371 of the top valve stem 37 does not abut the inner surface of the bottom annular member 42 of the adapter 40 to allow the interior of the upper compression tube 33 to communicate with the interior of the adapter 40.

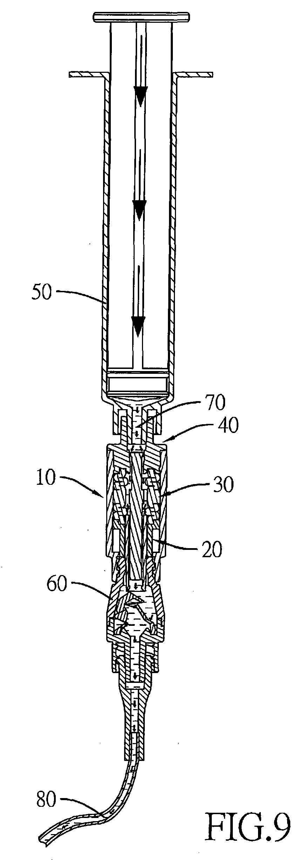

When the dosing unit 60 is screwed into the housing 10, the valve tube 12 of the housing 10 is inserted into the dosing unit 60 and compress a resilient member 61 of the dosing unit 60. Thus, the open bottom of the valve tube 12 communicates with an interior of the dosing unit 60 and a flow channel is formed within the needleless medical connector and the dosing unit 60. With reference to FIG. 9, medication 70 in the needleless medical injector 50 flows in sequence through the needleless medical connector, the dosing unit 60, and a fluid infusion tube 80 connected to the dosing unit 60, and the medication 70 is then injected into a patient.

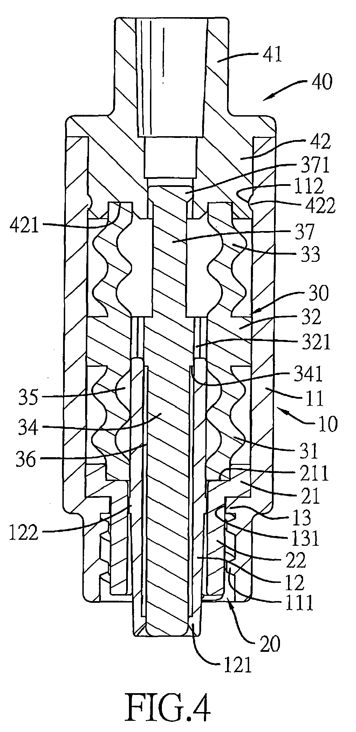

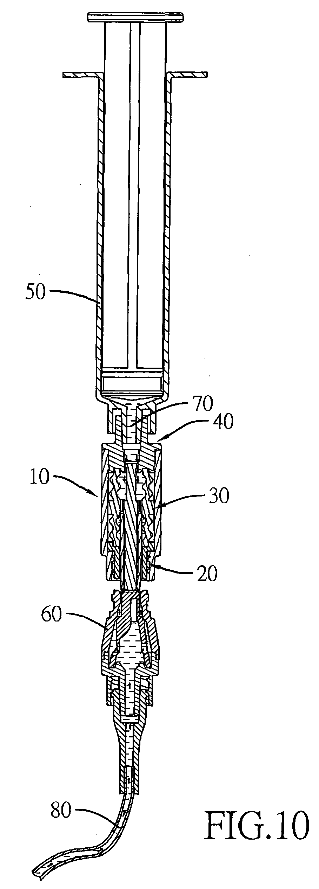

With reference to FIGS. 4 and 10, after the necessary medication has been dispensed, the dosing unit 60 is removed from the needleless medical connector. The lower and upper compression tubes 31, 33 extend themselves due to their resilience to produce three sealing effects as follows. Firstly, the bottom end of the bottom valve stem 34 returns to abut the bottom annular blocking projection 121 of the valve tube 12 of the housing 10 and to close the open bottom of the valve tube 12. Secondly, the flange 341 of the bottom valve stem 34 returns to be inserted into the top of the gap 36. Thirdly, the enlarged portion 371 of the top valve stem 37 returns to abut the inner surface of the bottom annular member 42 of the adapter 40. Therefore, the needleless medical connector has good sealing property to prevent leakage of the medication. In addition, the moment the dosing unit 60 is removed, a vacuum is formed in the needleless medical connector and draws the medication remaining on the external surface of the needleless medical connector into the needleless medical connector such that no medication residue exists outside the needleless medical connector.

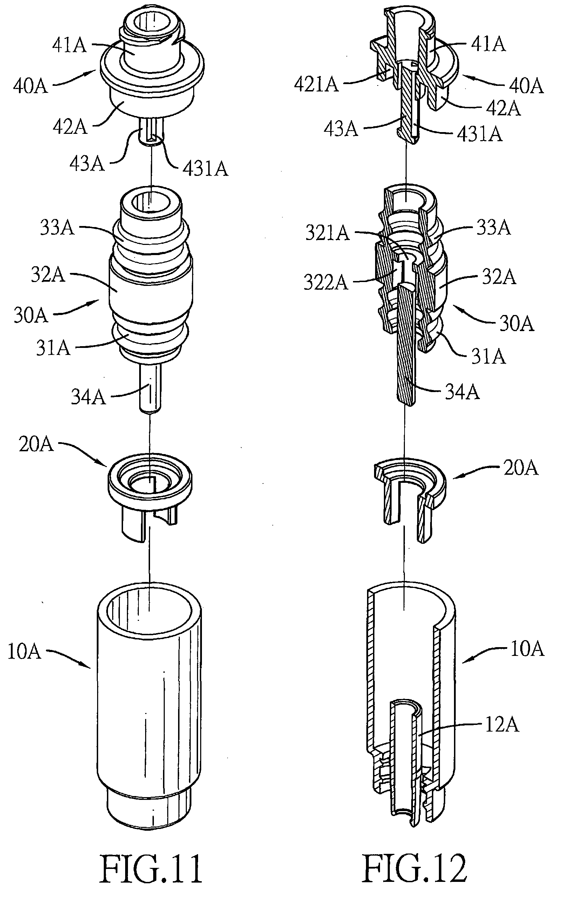

FIGS. 11 to 14 show a second embodiment of the leak proof needleless medical connector of the present invention.

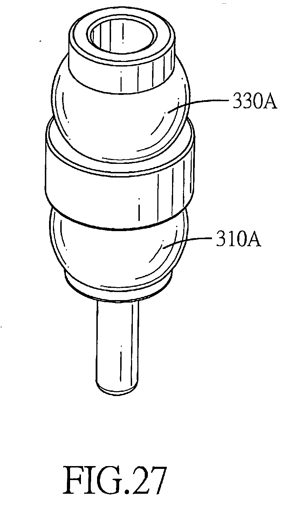

The column 32A has a through hole 321A extending through a central portion thereof. A top end of the bottom valve stem 34A is connected to a bottom end of an inner surface of the through hole 321A. A plurality of spaced longitudinal depressions 322A are formed in the inner surface of the through hole 321A. Each depression 322A has an open bottom communicating with the interior of the lower compression tube 31A and the gap 36A. Walls of the lower and upper compression tubes 31A, 33A may be waved, or, with reference to FIG. 27, walls of the lower and upper compression tubes 310A, 330A may be spherical.

The bottom annular member 42A of the adapter 40A has a bottom surface and a second annular receiving groove 421A. The second annular receiving groove 421A is formed in the bottom surface of the bottom annular member 42A. The top end of the upper compression tube 33A is received in the second annular receiving groove 421A. The top valve stem 43A extends downward from an interior of the bottom annular member 42A and has an outer surface and a plurality of spaced longitudinal channels 431A. The channels 431A are formed in the outer surface of the top valve stem 43A. Each channel 431A has an open top communicating with an interior of the top annular member 41A of the adapter 40A. A bottom end of the top valve stem 43A abuts a top end of the inner surface of the through hole 321A of the column 32A such that the interior of the upper compression tube 33A does not communicate with the through hole 321A of the column 32A.

In the second embodiment, the leak proof needleless medical connector comprises four components including the housing 10A, the actuator 20A, the resilient member 30A and the adapter 40A. The lower compression tube 31A, the column 32A, the upper compression tube 33A and the bottom valve stem 34A are integrally formed to construct the resilient member 30A. The top annular member 41A, the bottom annular member 42A and the top valve stem 43A are integrally formed to construct the adapter 40A.

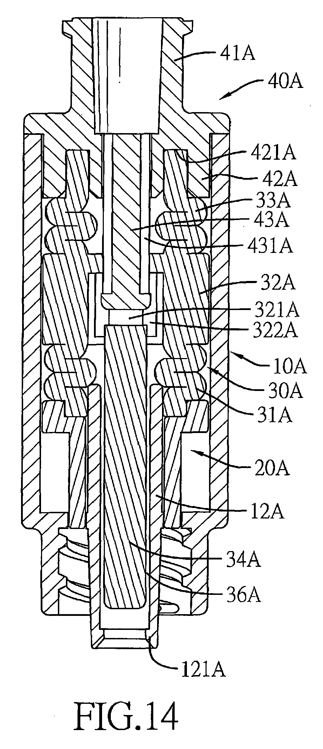

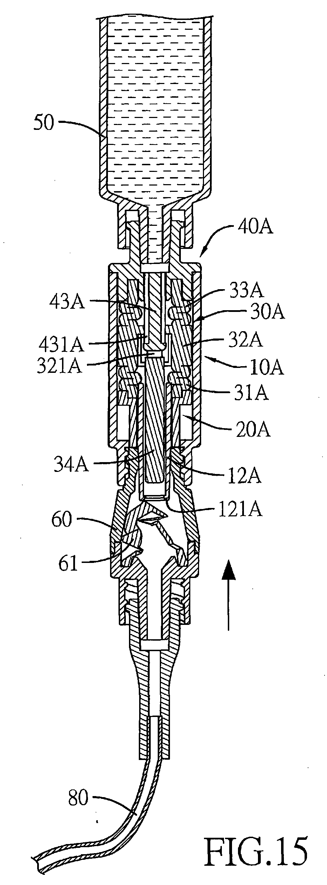

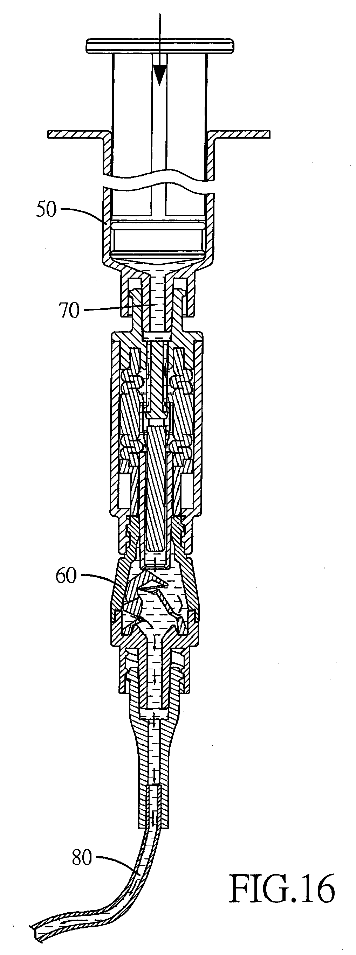

With reference to FIG. 7, when the second embodiment of the leak proof needleless medical connector is in use, the needleless medical connector is screwed into a needleless medical injector 50 and an appropriate dosing unit 60, 60A, 60B is then screwed into the needleless medical connector. The following description describes a situation in which the dosing unit 60 is used. With reference to FIGS. 14 and 15, the dosing unit 60 is screwed into the housing 10A to move the actuator 20A up and to compress the lower and upper compression tubes 31A, 33A. Under this circumstance, the bottom end of the bottom valve stem 34A is moved away from the bottom annular blocking projection 121A of the valve tube 12A of the housing 10A to open the open bottom of the valve tube 12A and to allow the open bottom of the valve tube 12A to communicate with an interior of the dosing unit 60, and the bottom end of the top valve stem 43A does not abut the inner surface of the through hole 321A of the column 32A to allow the interior of the upper compression tube 33A to communicate with the through hole 321A of the column 32A through the channels 431A of the top valve stem 43A. Thus, a flow channel is formed within the needleless medical connector and the dosing unit 60. With reference to FIG. 16, medication 70 in the needleless medical injector 50 flows in sequence through the needleless medical connector, the dosing unit 60, and a fluid infusion tube 80 connected to the dosing unit 60, and the medication is then injected into a patient.

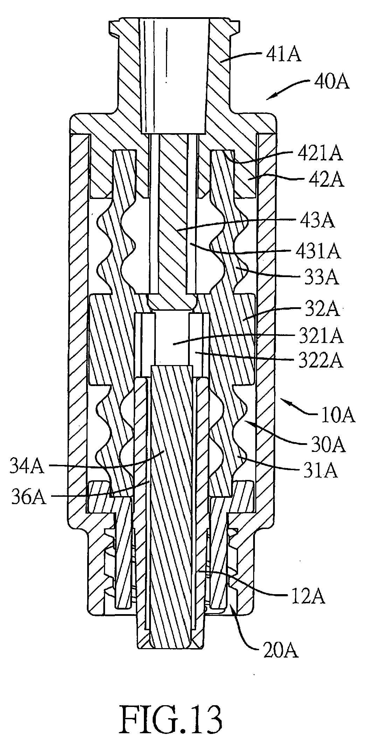

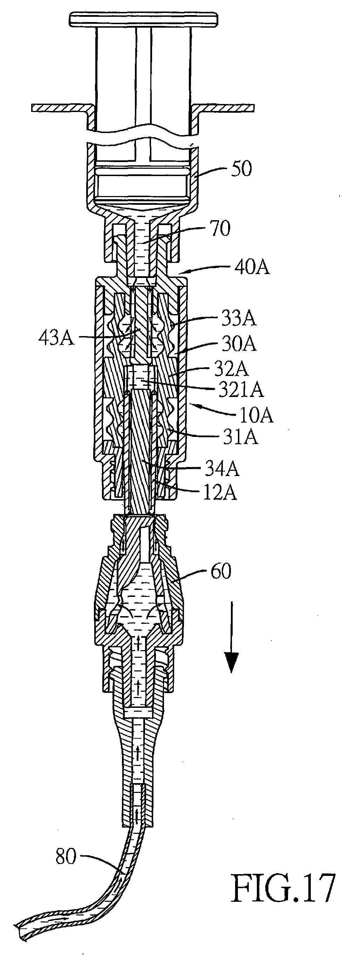

With reference to FIGS. 13 and 17, after the necessary medication has been dispensed, the dosing unit 60 is removed from the needleless medical connector. The lower and upper compression tubes 31A, 33A extend themselves due to their resilience to produce three sealing effects as follows. Firstly, the bottom end of the bottom valve stem 34A returns to close the open bottom of the valve tube 12A of the housing 10A. Secondly, the top end of the inner surface of the lower compression tube 31A returns to abut the outer surface of the valve tube 12A. Thirdly, the bottom end of the top valve stem 43A of the adapter 40A returns to abut the inner surface of the through hole 321A of the column 32A. In addition, the moment the dosing unit 60 is removed, a vacuum is formed in the needleless medical connector. Therefore, the needleless medical connector has good sealing property to prevent leakage of the medication and no medication residue exists outside the needleless medical connector.

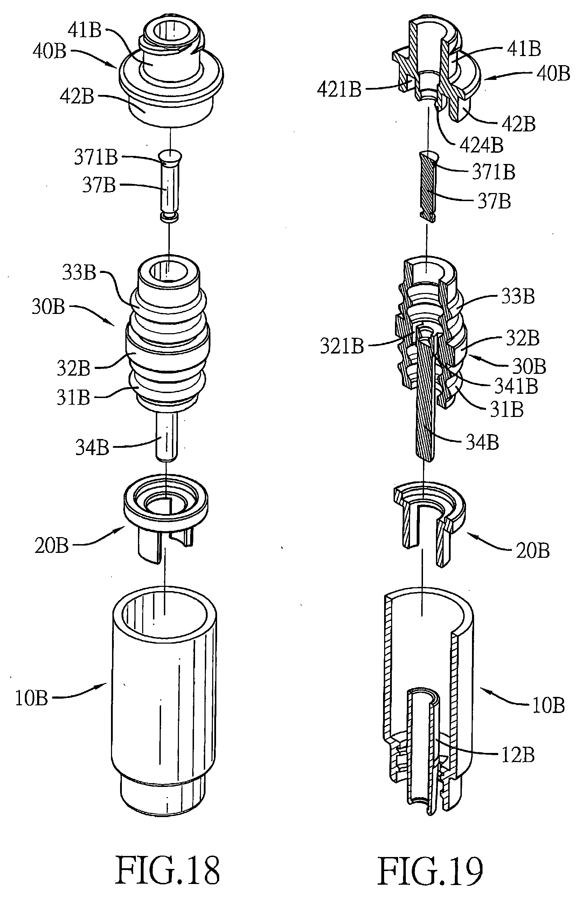

FIGS. 18 to 21 show a third embodiment of the leak proof needleless medical connector of the present invention.

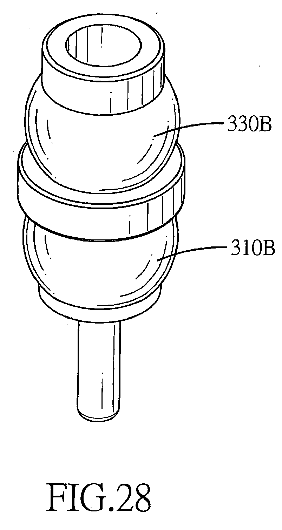

The bottom valve stem 34B extends downward from a central portion of a bottom surface of the column 32B. The column 32B has a plurality of through holes 321B therethrough. The through holes 321B are spaced around the bottom valve stem 34B. The bottom valve stem 34B has a flange 341B at its top end. The flange 341B is inserted into a top of the gap 36B such that the gap 36B does not communicate with the through holes 321B of the column 32B. The top valve stem 37B is mounted upward from a central portion of a top surface of the column 32B. The top valve stem 37B has an enlarged portion 371B at its top end. Walls of the lower and upper compression tubes 31B, 33B may be waved, or, with reference to FIG. 28, walls of the lower and upper compression tubes 310B, 330B may be spherical.

The bottom annular member 42B of the adapter 40B has a bottom surface, an inner surface, a second annular receiving groove 421B and a top annular blocking projection 424B. The second annular receiving groove 421B is formed in the bottom surface of the bottom annular member 42B. The top end of the upper compression tube 33B is received in the second annular receiving groove 421B. The top annular blocking projection 424B extends from a bottom end of the inner surface of the bottom annular member 42B. The enlarged portion 371B of the top valve stem 37B abuts the top annular blocking projection 424B such that the interior of the upper compression tube 33B does not communicate with the interior of the adapter 40B.

In the third embodiment, the leak proof needleless medical connector comprises five components including the housing 10B, the actuator 20B, the resilient member 30B, the top valve stem 37B and the adapter 40B. The lower compression tube 31B, the column 32B, the upper compression tube 33B and the bottom valve stem 34B are integrally formed to construct the resilient member 30B.

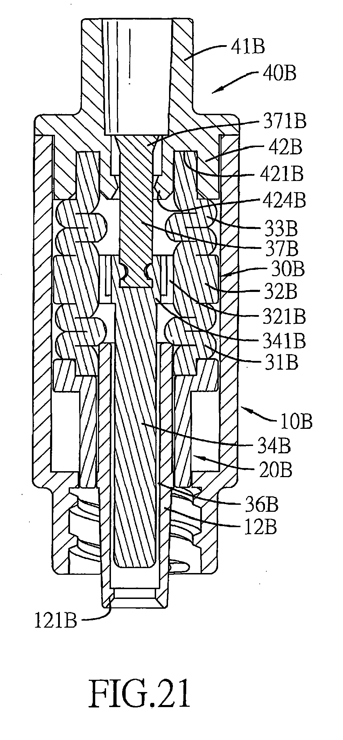

With reference to FIG. 7, when the third embodiment of the leak proof needleless medical connector is in use, the needleless medical connector is screwed into a needleless medical injector 50 and an appropriate dosing unit 60, 60A, 60B is then screwed into the needleless medical connector. The following description describes a situation in which the dosing unit 60 is used. With further reference to FIG. 21, the dosing unit 60 is screwed into the housing 10B to move the actuator 20B up and to compress the lower and upper compression tubes 31B, 33B. Under this circumstance, the bottom end of the bottom valve stem 34B is moved away from the bottom annular blocking projection 121B of the valve tube 12B of the housing 10B to open the open bottom of the valve tube 12B and to allow the open bottom of the valve tube 12B to communicate with an interior of the dosing unit 60, the flange 341B of the bottom valve stem 34B is moved away from the top of the gap 36B to allow the gap 36B to communicate with the through holes 321B of the column 32B, and the enlarged portion 371B of the top valve stem 37B is moved away from the top annular blocking projection 424B of the bottom annular member 42B of the adapter 40B to allow the interior of the upper compression tube 33B to communicate with the interior of the adapter 40B. Thus, a flow channel is formed within the needleless medical connector and the dosing unit 60 to allow passage of medication and injection of the medication into a patient.

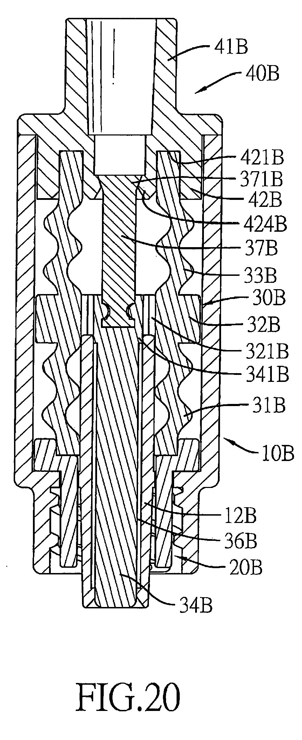

With reference to FIG. 20, after the necessary medication has been dispensed, the dosing unit 60 is removed from the needleless medical connector. The lower and upper compression tubes 31B, 33B extend themselves due to their resilience to produce three sealing effects as follows. Firstly, the bottom end of the bottom valve stem 34B returns to close the open bottom of the valve tube 12B of the housing 10B. Secondly, the flange 341B of the bottom valve stem 34B returns to be inserted into the top of the gap 36B. Thirdly, the enlarged portion 371B of the top valve stem 37B returns to abut the top annular blocking projection 424B of the bottom annular member 42B of the adapter 40B. In addition, the moment the dosing unit 60 is removed, a vacuum is formed in the needleless medical connector. Therefore, the needleless medical connector has good sealing property to prevent leakage of the medication and no medication residue exists outside the needleless medical connector.

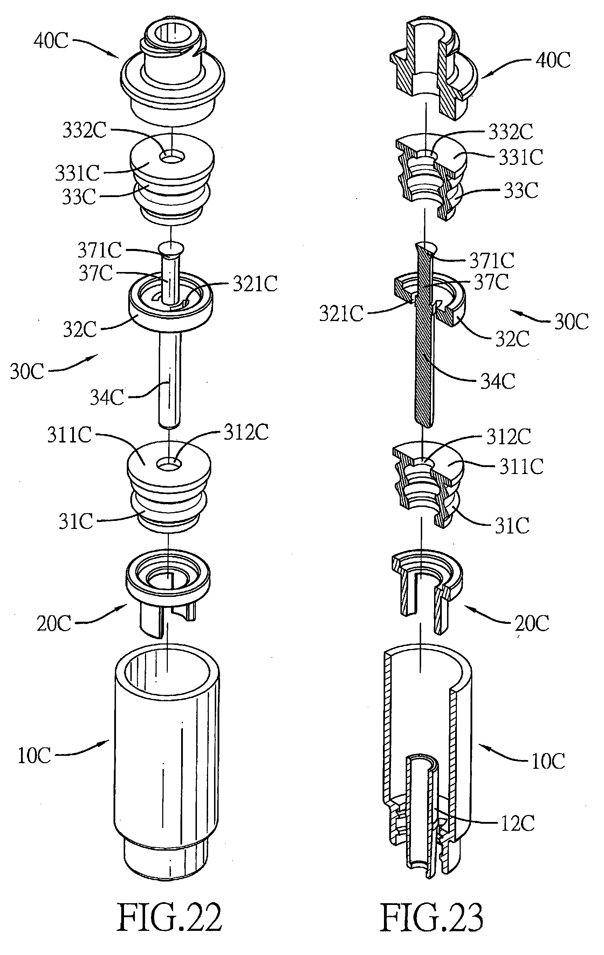

FIGS. 22 to 25 show a fourth embodiment of the leak proof needleless medical connector of the present invention.

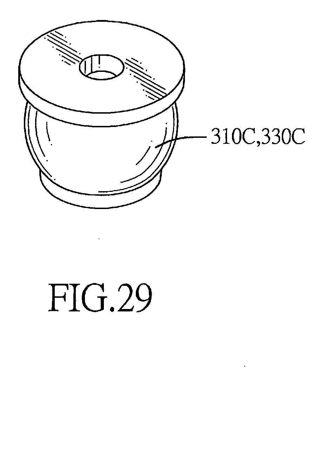

The lower compression tube 31C is mounted between the actuator 20C and the column 32C and has a lower top plate 311C and a lower central hole 312C. The lower central hole 312C extends through the lower top plate 311C. The upper compression tube 33C is mounted between the column 32C and the adapter 40C and has an upper top plate 331C and an upper central hole 332C. The upper central hole 332C extends through the upper top plate 331C. The bottom valve stem 34C extends downward from a central portion of a bottom surface of the column 32C. The top valve stem 37C extends upward from a central portion of a top surface of the column 32C. The column 32C has a plurality of through holes 321C therethrough. The through holes 321C are spaced around the bottom valve stem 34C and the top valve stem 37C. A top of the valve tube 12C of the housing 10C abuts a bottom surface of the lower top plate 311C of the lower compression tube 31C to urge the lower top plate 311C toward the column 32C to close open bottoms of the through holes 321C of the column 32C such that the through holes 321C do not communicate with the gap 36C. The top valve stem 37C has an enlarged portion 371C at its top end. The enlarged portion 371C abuts an inner surface of the upper central hole 332C of the upper compression tube 33C such that the interior of the upper compression tube 33C does not communicate with the interior of the adapter 40C. Walls of the lower and upper compression tubes 31C, 33C may be waved, or, with reference to FIG. 29, walls of the lower and upper compression tubes 310C, 330C may be spherical.

In the fourth embodiment, the leak proof needleless medical connector comprises six components including the housing 10C, the actuator 20C, the lower compression tube 31C, the column 32C, the upper compression tube 33C and the adapter 40C. The bottom valve stem 34C and the top valve stem 37C are integrally formed on the column 32C.

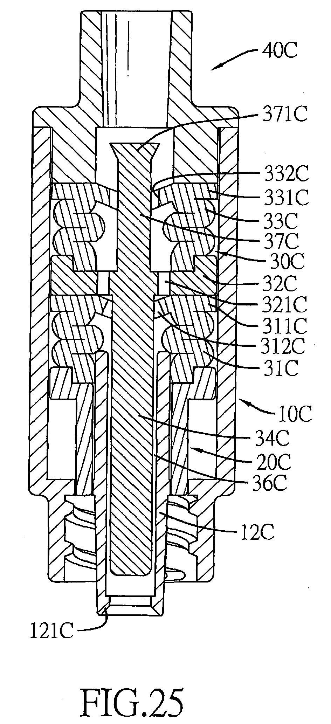

With reference to FIG. 7, when the fourth embodiment of the leak proof needleless medical connector is in use, the needleless medical connector is screwed into a needleless medical injector 50 and an appropriate dosing unit 60, 60A, 60B is then screwed into the needleless medical connector. The following description describes a situation in which the dosing unit 60 is used. With further reference to FIG. 25, the dosing unit 60 is screwed into the housing 10C to move the actuator 20C up and to compress the lower and upper compression tubes 31C, 33C. Under this circumstance, the bottom end of the bottom valve stem 34C is moved away from the bottom annular blocking projection 121C of the valve tube 12C of the housing 10C to open the open bottom of the valve tube 12C and to allow the open bottom of the valve tube 12C to communicate with an interior of the dosing unit 60, the top of the valve tube 12C does not abut the lower top plate 311C of the lower compression tube 31C to allow the gap 36C to communicate with the through holes 321C of the column 32C through the lower central hole 312C of the lower compression tube 31C, and the enlarged portion 371C of the top valve stem 37C does not abut the inner surface of the upper central hole 332C of the upper compression tube 33C to allow the interior of the upper compression tube 33C to communicate with the interior of the adapter 40C. Thus, a flow channel is formed within the needleless medical connector and the dosing unit 60 to allow passage of medication and injection of the medication into a patient.

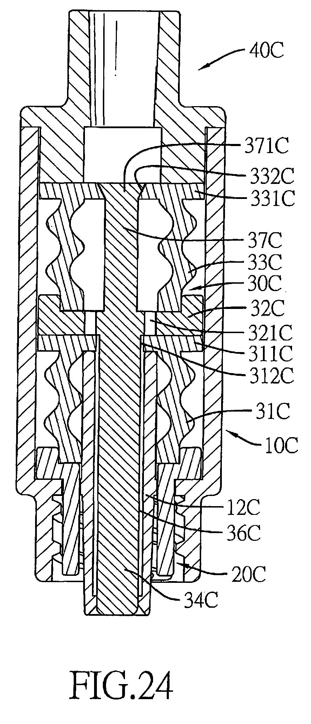

With reference to FIG. 24, after the necessary medication has been dispensed, the dosing unit 60 is removed from the needleless medical connector. The lower and upper compression tubes 31C, 33C extend themselves due to their resilience to produce three sealing effects as follows. Firstly, the bottom end of the bottom valve stem 34C returns to close the open bottom of the valve tube 12C of the housing 10C. Secondly, the top of the valve tube 12C returns to abut the lower top plate 311C of the lower compression tube 31C. Thirdly, the enlarged portion 371C of the top valve stem 37C returns to abut the inner surface of the upper central hole 332C of the upper compression tube 33C. In addition, the moment the dosing unit 60 is removed, a vacuum is formed in the needleless medical connector. Therefore, the needleless medical connector has good sealing property to prevent leakage of the medication and no medication residue exists outside the needleless medical connector.

## Claims

1. A leak proof needleless medical connector **characterized in that** the leak proof needleless medical connector comprises
a housing (10) including
an outer casing (11); and
a valve tube (12) disposed within the outer casing (11) and having
an inner surface; and
a bottom annular blocking projection (121) extending from a bottom end of the inner surface of the valve tube (12);
an actuator (20) being hollow, movably disposed within the outer casing (11) of the housing (10) and mounted around the valve tube (12) of the housing (10); **characterized in that** it further comprises
a resilient member (30) disposed within the outer casing (11) of the housing (10) and including in sequence
a lower compression tube (31) being resiliently compressible and expansible and mounted around the valve tube (12) of the housing (10);
a column (32) having at least one through hole (321) defined therethrough; and
an upper compression tube (33) being resiliently compressible and expansible, and an interior of the upper compression tube (33) communicating with an interior of the lower compression tube (31) through the at least one through hole (321) of the column (32);
a bottom valve stem (34) disposed in the lower compression tube (31) and mounted in the valve tube (12) of the housing (10), a gap (36) formed between the bottom valve stem (34) and the valve tube (12) and not communicating with the interior of the upper compression tube (33), a bottom end of the bottom valve stem (34) abutting the bottom annular blocking projection (121) of the valve tube (12) to close an open bottom of the valve tube (12);
a top valve stem (37) disposed in the upper compression tube (33); and
an adapter (40) being hollow and mounted on a top of the outer casing (11) of the housing (10), the resilient member (30) mounted between the adapter (40) and the actuator (20), and an interior of the adapter (40) not communicating with the at least one through hole (321) of the column (32) because of the top valve stem (37).

2. The leak proof needleless medical connector as claimed in claim 1, wherein
the housing (10) includes an annular portion (13) formed between the outer casing (11) and the valve tube (12) and having a plurality of guide holes (131) defined through the annular portion (13); and
the actuator (20) includes
a collar (21) having a bottom surface; and
a plurality of guide members (22) extending downward from the bottom surface of the collar (21) and corresponding to and inserted respectively through the guide holes (131) of the annular portion (13) of the housing (10).

3. The leak proof needleless medical connector as claimed in claim 2, wherein the adapter (40) includes
a top annular member (41); and
a bottom annular member (42) inserted into the top of the outer casing (11) of the housing (10).

4. The leak proof needleless medical connector as claimed in claim 3, wherein the bottom valve stem (34) extends downward from a bottom surface of the column (32), the top valve stem (37) extends upward from a top surface of the column (32), the column (32) has a plurality of through holes (321) defined therethrough, the through holes (321) are spaced around the bottom valve stem (34) and the top valve stem (37), the bottom valve stem (34) has a flange (341) at its top end, the flange (341) is inserted into a top of the gap (36) such that the gap (36) does not communicate with the through holes (321) of the column (32), the top valve stem (37) has an enlarged portion (371) at its top end, and the enlarged portion (371) abuts an inner surface of the bottom annular member (42) of the adapter (40) such that the interior of the upper compression tube (33) does not communicate with the interior of the adapter (40).

5. The leak proof needleless medical connector as claimed in claim 4 comprising four components including the housing (10), the actuator (20), the resilient member (30) and the adapter (40), wherein the lower compression tube (31), the column (32), the upper compression tube (33), the bottom valve stem (34) and the top valve stem (37) are integrally formed to construct the resilient member (30).

6. The leak proof needleless medical connector as claimed in claim 5, wherein
the outer casing (11) of the housing (10) has
an inner surface; and
an annular locking projection (112) and a plurality of spaced longitudinal locking ribs (113) extending from the inner surface of the outer casing (11); and
the bottom annular member (42) of the adapter (40) has
an outer surface; and
an annular locking groove (422) and a plurality of spaced longitudinal locking channels (423) formed in the outer surface of the bottom annular member (42), the annular locking projection (112) of the outer casing (11) of the housing (10) corresponding to and engaging the annular locking groove (422), and the longitudinal locking ribs (113) of the outer casing (11) of the housing (10) corresponding to and engaging the longitudinal locking channels (423).

7. The leak proof needleless medical connector as claimed in claim 3, wherein
the column (32A) has a through hole (321A) defined therethrough, a top end of the bottom valve stem (34A) is connected to a bottom end of an inner surface of the through hole (321A), a plurality of depressions (322A) are formed in the inner surface of the through hole (321A), and each depression (322A) has an open bottom communicating with the interior of the lower compression tube (31A) and the gap (36A);
the top valve stem (43A) extends downward from an interior of the bottom annular member (42A) of the adapter (40A) and has
an outer surface; and
a plurality of channels (431A) formed in the outer surface of the top valve stem (43A), each channel (431A) having an open top communicating with an interior of the top annular member (41A) of the adapter (40A), and
a bottom end of the top valve stem (43A) abuts the inner surface of the through hole (321A) of the column (32A) such that the interior of the upper compression tube (33A) does not communicate with the through hole (321A) of the column (32A).

8. The leak proof needleless medical connector as claimed in claim 7 comprising four components including the housing (10A), the actuator (20A), the resilient member (30A) and the adapter (40A), wherein the lower compression tube (31A), the column (32A), the upper compression tube (33A) and the bottom valve stem (34A) are integrally formed to construct the resilient member (30A), and wherein the top annular member (41A), the bottom annular member (42A) and the top valve stem (43A) are integrally formed to construct the adapter (40A).

9. The leak proof needleless medical connector as claimed in claim 3, wherein
the bottom valve stem (34B) extends downward from a bottom surface of the column (32B), the column (32B) has a plurality of through holes (321B) defined therethrough, the through holes (321B) are spaced around the bottom valve stem (34B), the bottom valve stem (34B) has a flange (341B) at its top end, the flange (341B) is inserted into a top of the gap (36B) such that the gap (36B) does not communicate with the through holes (321B) of the column (32B);
the bottom annular member (42B) of the adapter (40B) has
an inner surface; and
a top annular blocking projection (424B) extending from the inner surface of the bottom annular member (42B), and
the top valve stem (37B) is mounted upward from a top surface of the column (32B) and has an enlarged portion (371B) at its top end, and the enlarged portion (371B) abuts the top annular blocking projection (424B) of the bottom annular member (42B) of the adapter (40B) such that the interior of the upper compression tube (33B) does not communicate with the interior of the adapter (40B).

10. The leak proof needleless medical connector as claimed in claim 9 comprising five components including the housing (10B), the actuator (20B), the resilient member (30B), the top valve stem (37B) and the adapter (40B), wherein the lower compression tube (31B), the column (32B), the upper compression tube (33B) and the bottom valve stem (34B) are integrally formed to construct the resilient member (30B).

11. The leak proof needleless medical connector as claimed in claim 3, wherein
the lower compression tube (31C) has
a lower top plate (311 C); and
a lower central hole (312C) extending through the lower top plate (311C);
the upper compression tube (33C) has
an upper top plate (331C); and
an upper central hole (332C) extending through the upper top plate (331C); and
the bottom valve stem (34C) extends downward from a bottom surface of the column (32C), the top valve stem (37C) extends upward from a top surface of the column (32C), the column (32C) has a plurality of through holes (321C) defined therethrough, the through holes (321C) are spaced around the bottom valve stem (34C) and the top valve stem (37C), a top of the valve tube (12C) of the housing (10C) abuts a bottom surface of the lower top plate (311 C) of the lower compression tube (31 C) to urge the lower top plate (311 C) toward the column (32C) to close open bottoms of the through holes (321C) of the column (32C) such that the through holes (321C) do not communicate with the gap (36C), the top valve stem (37C) has an enlarged portion (371C) at its top end, and the enlarged portion (371C) abuts an inner surface of the upper central hole (332C) of the upper compression tube (33C) such that the interior of the upper compression tube (33C) does not communicate with the interior of the adapter (40C).

12. The leak proof needleless medical connector as claimed in claim 11 comprising six components including the housing (10C), the actuator (20C), the lower compression tube (31C), the column (32C), the upper compression tube (33C) and the adapter (40C), wherein the bottom valve stem (34C) and the top valve stem (37C) are integrally formed on the column (32C).

13. The leak proof needleless medical connector as claimed in any one of claims 1 to 12, wherein walls of the lower and upper compression tubes (31, 33, 31A, 33A, 31B, 33B, 31C, 33C) are waved.

14. The leak proof needleless medical connector as claimed in any one of claims 1 to 12, wherein walls of the lower and upper compression tubes (310, 330, 310A, 330A, 310B, 330B, 310C, 330C) are spherical.

## Patentansprüche

1. Auslaufsicherer nadelloser medizinischer Verbinder, **dadurch gekennzeichnet, dass** der auslaufsichere nadellose medizinische Verbinder Folgendes umfasst:
ein Gehäuse (10), das Folgendes einschließt:
eine äußere Hülle (11) und
eine Ventilröhre (12), die innerhalb der äußeren Hülle (11) angeordnet ist und Folgendes aufweist:
eine Innenfläche und
einen unteren ringförmigen Blockiervorsprung (121), der sich von einem unteren Ende der Innenfläche der Ventilröhre (12) aus erstreckt,
einen Stellantrieb (20), der hohl, beweglich innerhalb der äußeren Hülle (11) des Gehäuses (10) angeordnet und um die Ventilröhre (12) des Gehäuses (10) angeordnet ist,
**dadurch gekennzeichnet, dass** er ferner Folgendes umfasst:
ein elastisches Element (30), das innerhalb der äußeren Hülle (11) des Gehäuses (10) angeordnet ist und der Reihe nach Folgendes einschließt:
eine untere Kompressionsröhre (31), die elastisch komprimierbar und expandierbar und um die Ventilröhre (12) des Gehäuses (10) angeordnet ist,
eine Säule (32), die wenigstens ein Durchgangsloch (321) hat, das durch dieselbe definiert ist, und
eine obere Kompressionsröhre (33), die elastisch komprimierbar und expandierbar ist und wobei ein Inneres der oberen Kompressionsröhre (33) mit einem Inneren der unteren Kompressionsröhre (31) durch das wenigstens eine Durchgangsloch (321) der Säule (32) in Verbindung steht,
einen unteren Ventilschaft (34), der in der unteren Kompressionsröhre (31) angeordnet und in der Ventilröhre (12) des Gehäuses (10) angebracht ist, wobei ein Spalt (36) zwischen dem unteren Ventilschaft (34) und der Ventilröhre (12) geformt ist und nicht mit dem inneren der oberen Kompressionsröhre (33) in Verbindung steht, wobei ein unteres Ende des unteren Ventilschafts (34) an den unteren ringförmigen Blockiervorsprung (121) der Ventilröhre (12) anstößt, um einen offenen Unterteil der Ventilröhre (12) zu schließen,
einen oberen Ventilschaft (37) der in der oberen Kompressionsröhre (33) angeordnet ist, und
einen Adapter (40), der hohl und an einem Oberteil der äußeren Hülle (11) des Gehäuses (10) angebracht ist, wobei das elastische Element (30) zwischen dem Adapter (40) und dem Stellantrieb (20) angebracht ist und ein Inneres des Adapters (40) wegen des oberen Ventilschafts (37) nicht mit dem wenigstens einen Durchgangsloch (321) der Säule (32) in Verbindung steht.

2. Auslaufsicherer nadelloser medizinischer Verbinder nach Anspruch 1, wobei
das Gehäuse (10) einen ringförmigen Abschnitt (13) einschließt, der zwischen der äußeren Hülle (11) und der Ventilröhre (12) geformt ist und mehrere Führungslöcher (131) hat, die durch den ringförmigen Abschnitt (13) definiert sind, und
der Stellantrieb (20) Folgendes einschließt:
einen Bund (21), der eine untere Fläche hat, und
mehrere Führungselemente (22), die sich von der unteren Fläche des Bundes (21) aus nach unten erstrecken und jeweils den Führungslöchern (131) des ringförmigen Abschnitts (13) des Gehäuses (10) entsprechen und durch dieselben eingesetzt werden.

3. Auslaufsicherer nadelloser medizinischer Verbinder nach Anspruch 2, wobei der Adapter (40) Folgendes einschließt:
ein oberes ringförmiges Element (41) und
ein unteres ringförmiges Element (42), das in den Oberteil der äußeren Hülle (11) des Gehäuses (10) eingesetzt wird.

4. Auslaufsicherer nadelloser medizinischer Verbinder nach Anspruch 3, wobei sich der untere Ventilschaft (34) von einer unteren Fläche der Säule (32) aus nach unten erstreckt, sich der obere Ventilschaft (37) von einer oberen Fläche der Säule (32) aus nach oben erstreckt, die Säule (32) mehrere Durchgangslöcher (321) hat, die durch dieselbe definiert sind, die Durchgangslöcher (321) um den unteren Ventilschaft (34) und den oberen Ventilschaft (37) beabstandet sind, der untere Ventilschaft (34) einen Flansch (341) an seinem oberen Ende hat, der Flansch (341) derart in einen Oberteil des Spalts (36) eingesetzt wird, dass der Spalt (36) nicht mit den Durchgangslöchern (321) der Säule (32) in Verbindung steht, der obere Ventilschaft (37) einen vergrößerten Abschnitt (371) an seinem oberen Ende hat und der vergrößerte Abschnitt (371) derart an eine Innenfläche des unteren ringförmigen Elements (42) des Adapters (40) anstößt, dass das Innere der oberen Kompressionsröhre (33) nicht mit dem Inneren des Adapters (40) in Verbindung steht.

5. Auslaufsicherer nadelloser medizinischer Verbinder nach Anspruch 4, der vier Bestandteile umfasst, die das Gehäuse (10), den Stellantrieb (20), das elastische Element (30) und den Adapter (40) einschließen, wobei die untere Kompressionsröhre (31), die Säule (32), die obere Kompressionsröhre (33), der untere Ventilschaft (34) und der obere Ventilschaft (37) integral geformt sind, um das elastische Element (30) aufzubauen.

6. Auslaufsicherer nadelloser medizinischer Verbinder nach Anspruch 5, wobei
die äußere Hülle (11) des Gehäuses (10) Folgendes aufweist:
eine Innenfläche und
einen ringförmigen Verriegelungsvorsprung (112) und mehrere beabstandete längsgerichtete Verriegelungsrippen (113), die sich von der Innenfläche der äußeren Hülle (11) aus erstrecken, und
das untere ringförmige Element (42) des Adapters (40) Folgendes aufweist:
eine Außenfläche und
eine ringförmige Verriegelungsrille (422) und mehrere beabstandete längsgerichtete Verriegelungskanäle (423), die in der Außenfläche des unteren ringförmigen Elements (42) geformt sind, wobei der ringförmige Verriegelungsvorsprung (112) der äußeren Hülle (11) des Gehäuses (10) der ringförmigen Verriegelungsrille (422) entspricht und dieselbe in Eingriff nimmt und die längsgerichteten Verriegelungsrippen (113) der äußeren Hülle (11) des Gehäuses (10) den längsgerichteten Verriegelungskanälen (423) entsprechen und dieselben in Eingriff nehmen.

7. Auslaufsicherer nadelloser medizinischer Verbinder nach Anspruch 3, wobei
die Säule (32A) ein Durchgangsloch (321A) hat, das durch dieselbe definiert ist, ein oberes Ende des unteren Ventilschafts (34A) mit einem unteren Ende einer Innenfläche des Durchgangslochs (321A) verbunden ist, mehrere Vertiefungen (322A) in der Innenfläche des Durchgangslochs (321A) Innenfläche des Durchgangslochs geformt sind und jede Vertiefung (322A) einen offenen Unterteil hat, der mit dem Inneren der unteren Kompressionsröhre (31A) und dem Spalt (36A) in Verbindung steht,
sich der obere Ventilschaft (43A) von einem Inneren des unteren ringförmigen Elements (42A) des Adapters (40A) aus nach unten erstreckt und Folgendes aufweist:
eine Außenfläche und
mehrere Kanäle (431A), die in der Außenfläche des oberen Ventilschafts (43A) geformt sind, wobei jeder Kanal (431A) einen offenen Oberteil hat, der mit einem Inneren des oberen ringförmigen Elements (41A) des Adapters (40A) in Verbindung steht, und
ein unteres Ende des oberen Ventilschafts (43A) derart an die Innenfläche des Durchgangslochs (321A) der Säule (32A) anstößt, dass das Innere der oberen Kompressionsröhre (33A) nicht mit dem Durchgangsloch (321A) der Säule (32A) in Verbindung steht.

8. Auslaufsicherer nadelloser medizinischer Verbinder nach Anspruch 7, der vier Bestandteile umfasst, die das Gehäuse (10A), den Stellantrieb (20A), das elastische Element (30A) und den Adapter (40A) einschließen, wobei die untere Kompressionsröhre (31A), die Säule (32A), die obere Kompressionsröhre (33A) und der untere Ventilschaft (34A) integral geformt sind, um das elastische Element (30A) aufzubauen, und wobei das obere ringförmige Element (41A), das untere ringförmige Element (42A) und der obere Ventilschaft (43A) integral geformt sind, um den Adapter (40A) aufzubauen.

9. Auslaufsicherer nadelloser medizinischer Verbinder nach Anspruch 3, wobei
sich der untere Ventilschaft (34B) von einer unteren Fläche der Säule (32B) aus nach unten erstreckt, die Säule (32B) mehrere Durchgangslöcher (321B) hat, die durch dieselbe definiert sind, die Durchgangslöcher (321B) um den unteren Ventilschaft (34B) beabstandet sind, der untere Ventilschaft (34B) einen Flansch (341B) an seinem oberen Ende hat, der Flansch (341B) derart in einen Oberteil des Spalts (36B) eingesetzt wird, dass der Spalt (36B) nicht mit den Durchgangslöchern (321B) der Säule (32B) in Verbindung steht,
das untere ringförmige Element (42B) des Adapters (40B) Folgendes aufweist:
eine Innenfläche und
einen oberen ringförmigen Blockiervorsprung (424B), der sich von der Innenfläche des unteren ringförmigen Elements (42B) aus erstreckt, und
der obere Ventilschaft (37B) von einer oberen Fläche der Säule (32B) aus nach oben angebracht ist und einen vergrößerten Abschnitt (371B) an seinem oberen Ende hat und der vergrößerte Abschnitt (371B) derart an den oberen ringförmigen Blockiervorsprung (424B) des unteren ringförmigen Elements (42B) des Adapters (40B) anstößt, dass das Innere der oberen Kompressionsröhre (33B) nicht mit dem Inneren des Adapters (40B) in Verbindung steht.

10. Auslaufsicherer nadelloser medizinischer Verbinder nach Anspruch 9, der fünf Bestandteile umfasst, die das Gehäuse (10B), den Stellantrieb (20B), das elastische Element (30B), den oberen Ventilschaft (37B) und den Adapter (40B) einschließen, wobei die untere Kompressionsröhre (31B), die Säule (32B), die obere Kompressionsröhre (33B) und der untere Ventilschaft (34B) integral geformt sind, um das elastische Element (30B) aufzubauen.

11. Auslaufsicherer nadelloser medizinischer Verbinder nach Anspruch 3, wobei
die untere Kompressionsröhre (31C) Folgendes aufweist:
eine untere Deckplatte (311C) und
ein unteres Mittelloch (312C), das sich durch die untere Deckplatte (311C) erstreckt,
die obere Kompressionsröhre (33C) Folgendes aufweist:
eine obere Deckplatte (331C) und
ein oberes Mittelloch (332C), das sich durch die obere Deckplatte (331C) erstreckt, und
sich der untere Ventilschaft (34C) von einer unteren Fläche der Säule (32C) aus nach unten erstreckt, sich der obere Ventilschaft (37C) von einer oberen Fläche der Säule (32C) aus nach oben erstreckt, die Säule (32C) mehrere Durchgangslöcher (321C) hat, die durch dieselbe definiert sind, die Durchgangslöcher (321C) um den unteren Ventilschaft (34C) und den oberen Ventilschaft (37C) beabstandet sind, ein Oberteil der Ventilröhre (12C) des Gehäuses (10C) an eine untere Fläche der unteren Deckplatte (311C) der unteren Kompressionsröhre (31C) anstößt, um die untere Deckplatte (311C) zu der Säule (32C) hin zu drängen, um offene Unterteile der Durchgangslöcher (321C) der Säule (32C) derart zu schließen, dass die Durchgangslöcher (321C) nicht in Verbindung mit dem Spalt (36C) stehen, der obere Ventilschaft (37C) einen vergrößerten Abschnitt (371C) an seinem oberen Ende hat und der vergrößerte Abschnitt (371C) derart an eine Innenfläche des oberen Mittellochs (332C) der oberen Kompressionsröhre (33C) anstößt, dass das Innere der oberen Kompressionsröhre (33C) nicht mit dem Inneren des Adapters (40C) in Verbindung steht.

12. Auslaufsicherer nadelloser medizinischer Verbinder nach Anspruch 11, der sechs Bestandteile umfasst, die das Gehäuse (10C) den Stellantrieb (20C), die untere Kompressionsröhre (31C), die Säule (32C), die obere Kompressionsröhre (33C) und den Adapter (40C) einschließen, wobei der untere Ventilschaft (34C) und der obere Ventilschaft (37C) integral an der Säule (32C) geformt sind.

13. Auslaufsicherer nadelloser medizinischer Verbinder nach einem der Ansprüche 1 bis 12, wobei Wände der unteren und der oberen Kompressionsröhren (31, 33, 31A, 33A, 31B, 33B, 31C, 33C) gewellt sind.

14. Auslaufsicherer nadelloser medizinischer Verbinder nach einem der Ansprüche 1 bis 12, wobei Wände der unteren und der oberen Kompressionsröhren (310, 330, 310A, 330A, 310B, 330B, 310C, 330C) sphärisch sind.

## Revendications

1. Connecteur médical étanche sans aiguille, **caractérisé en ce que**
le connecteur médical étanche sans aiguille comprend
un boîtier (10) comprenant
une enveloppe extérieure (11) ; et
un tube de soupape (12) disposé à l'intérieur de l'enveloppe extérieure (11) et comportant
une surface intérieure ; et
une saillie de blocage annulaire inférieure (121) s'étendant à partir d'une extrémité inférieure de la surface intérieure du tube de soupape (12) ;
un dispositif d'actionnement (20) creux, disposé de façon mobile à l'intérieur de l'enveloppe extérieure (11) du boîtier (10) et monté autour du tube de soupape (12) du boîtier (10) ;
**caractérisé en ce qu'**il comprend en outre
un élément élastique (30) disposé à l'intérieur de l'enveloppe extérieure (11) du boîtier (10) et comportant successivement
un tube de compression inférieur (31) étant élastiquement compressible et expansible et monté autour du tube de soupape (12) du boîtier (10) ;
une colonne (32) comportant au moins un orifice de passage (321) défini à travers celle-ci ; et
un tube de compression supérieur (33) étant élastiquement compressible et expansible, et l'intérieur du tube de compression supérieur (33) communiquant avec l'intérieur du tube de compression inférieur (31) à travers le au moins un orifice de passage (321) de la colonne (32) ; et
une tige de soupape inférieure (34) disposée dans le tube de compression inférieur (31) et montée dans le tube de soupape (12) du boîtier (10), un espace (36) formé entre la tige de soupape inférieure (34) et le tube de soupape (12) et ne communiquant pas avec l'intérieur du tube de compression supérieur (33), une extrémité inférieure de la tige de soupape inférieure (34) portant contre la saillie de blocage annulaire inférieure (121) du tube de soupape (12) de manière à fermer une partie inférieure ouverte du tube de soupape (12) ;
une tige de soupape supérieure (37) disposée dans le tube de compression supérieur (33) ; et
un adaptateur (40) creux et monté sur une partie supérieure de l'enveloppe extérieure (11) du boîtier (10), l'élément élastique (30) monté entre l'adaptateur (40) et le dispositif d'actionnement (20), et l'intérieur de l'adaptateur (40) ne communiquant pas avec le au moins un orifice de passage (321) de la colonne (32) en raison de la tige de soupape supérieure (37).

2. Connecteur médical étanche sans aiguille selon la revendication 1, dans lequel
le boîtier (10) comprend une partie annulaire (13) formée entre l'enveloppe extérieure (11) et le tube de soupape (12) et comportant une pluralité de trous de guidage (131) définis à travers la partie annulaire (13) ; et
le dispositif d'actionnement (20) comprend
un épaulement (21) comportant une surface inférieure ; et
une pluralité d'éléments de guidage (22) s'étendant vers le bas à partir de la surface inférieure de l'épaulement (21) et correspondant aux trous de guidage (131) de la partie annulaire (13) du boîtier (10) et insérés respectivement à travers ceux-ci.

3. Connecteur médical étanche sans aiguille selon la revendication 2, dans lequel l'adaptateur (40) comprend
un élément annulaire supérieur (41) ; et
un élément annulaire inférieur (42) inséré dans la partie supérieure de l'enveloppe extérieure (11) du boîtier (10).

4. Connecteur médical étanche sans aiguille selon la revendication 3, dans lequel la tige de soupape inférieure (34) s'étend vers le bas depuis une surface inférieure de la colonne (32), la tige de soupape supérieure (37) s'étend vers le haut depuis une surface supérieure de la colonne (32), la colonne (32) comporte une pluralité d'orifices de passage (321) définis à travers celle-ci, les orifices de passage (321) sont espacés autour de la tige de soupape inférieure (34) et de la tige de soupape supérieure (37), la tige de soupape inférieure (34) comporte une bride (341) au niveau de son extrémité supérieure, la bride (341) est insérée dans le haut de l'espace (36) de telle sorte que l'espace (36) ne communique pas avec les orifices de passage (321) de la colonne (32), la tige de soupape supérieure (37) comporte une partie élargie (371) au niveau de son extrémité supérieure, et la partie élargie (371) porte contre une surface intérieure de l'élément annulaire inférieur (42) de l'adaptateur (40) de telle sorte que l'intérieur du tube de compression supérieur (33) ne communique pas avec l'intérieur de l'adaptateur (40).

5. Connecteur médical étanche sans aiguille selon la revendication 4, comprenant quatre composants incluant le boîtier (10), le dispositif d'actionnement (20), l'élément élastique (30) et l'adaptateur (40), dans lequel le tube de compression inférieur (31), la colonne (32), le tube de compression supérieur (33), la tige de soupape inférieure (34) et la tige de soupape supérieure (37) sont formés d'un seul tenant afin de composer l'élément élastique (30).

6. Connecteur médical étanche sans aiguille selon la revendication 5, dans lequel
l'enveloppe extérieure (11) du boîtier (10) comporte
une surface intérieure ; et
une saillie de blocage annulaire (112) et une pluralité de nervures de blocage longitudinales espacées (113) s'étendant depuis la surface intérieure de l'enveloppe extérieure (11) ; et
l'élément annulaire inférieur (42) de l'adaptateur (40) comporte
une surface extérieure ; et
une rainure de blocage annulaire (422) et une pluralité de canaux de blocage longitudinaux espacés (423) formés dans la surface extérieure de l'élément annulaire inférieur (42), la saillie de blocage annulaire (112) de l'enveloppe extérieure (11) du boîtier (10) correspondant à la rainure de blocage annulaire (422) et coopérant avec celle-ci, et les nervures de blocage longitudinales (113) de l'enveloppe extérieure (11) du boîtier (10) correspondant aux canaux de blocage longitudinaux (423) et coopérant avec ceux-ci.

7. Connecteur médical étanche sans aiguille selon la revendication 3, dans lequel
la colonne (32A) comporte un orifice de passage (321A) défini à travers celle-ci, une extrémité supérieure de la tige de soupape inférieure (34A) est reliée à une extrémité inférieure d'une surface intérieure de l'orifice de passage (321A), une pluralité de dépressions (322A) sont formées dans la surface intérieure de l'orifice de passage (321A), et chaque dépression (322A) comporte une partie inférieure ouverte communiquant avec l'intérieur du tube de compression inférieur (31A) et l'espace (36A) ;
la tige de soupape supérieure (43A) s'étend vers le bas depuis l'intérieur de l'élément annulaire inférieur (42A) de l'adaptateur (40A) et comporte
une surface extérieure ; et
une pluralité de canaux (431A) formés dans la surface extérieure de la tige de soupape supérieure (43A), chaque canal (431A) comportant une partie supérieure ouverte communiquant avec l'intérieur de l'élément annulaire supérieur (41A) de l'adaptateur (40A), et
une extrémité inférieure de la tige de soupape supérieure (43A) porte contre la surface intérieure de l'orifice de passage (321A) de la colonne (32A) de telle sorte que l'intérieur du tube de compression supérieur (33A) ne communique pas avec l'orifice de passage (321A) de la colonne (32A).

8. Connecteur médical étanche sans aiguille selon la revendication 7, comprenant quatre composants incluant le boîtier (10A), le dispositif d'actionnement (20A), l'élément élastique (30A) et l'adaptateur (40A), dans lequel le tube de compression inférieur (31A), la colonne (32A), le tube de compression supérieur (33A) et la tige de soupape inférieure (34A) sont formés d'un seul tenant pour composer l'élément élastique (30A), et dans lequel l'élément annulaire supérieur (41A), l'élément annulaire inférieur (42A) et la tige de soupape supérieure (43A) sont formés d'un seul tenant afin de composer l'adaptateur (40A).

9. Connecteur médical étanche sans aiguille selon la revendication 3, dans lequel
la tige de soupape inférieure (34B) s'étend vers le bas depuis une surface inférieure de la colonne (32B), la colonne (32B) comporte une pluralité d'orifices de passage (321B) définis à travers celle-ci, les orifices de passage (321B) sont espacés autour de la tige de soupape inférieure (34B), la tige de soupape inférieure (34B) comporte une bride (341B) au niveau de son extrémité supérieure, la bride (341B) est insérée dans le haut de l'espace (36B) de telle sorte que l'espace (36B) ne communique pas avec les orifices de passage (321B) de la colonne (32B) ;
l'élément annulaire inférieur (42B) de l'adaptateur (40B) comporte
une surface intérieure ; et
une saillie de blocage annulaire supérieure (424B) s'étendant depuis la surface intérieure de l'élément annulaire inférieur (42B), et
la tige de soupape supérieure (37B) est montée vers le haut depuis une surface supérieure de la colonne (32B) et comporte une partie élargie (371B) au niveau de son extrémité supérieure, et la partie élargie (371B) porte contre la saillie de blocage annulaire supérieure (424B) de l'élément annulaire inférieur (42B) de l'adaptateur (40B) de telle sorte que l'intérieur du tube de compression supérieur (33B) ne communique pas avec l'intérieur de l'adaptateur (40B).

10. Connecteur médical étanche sans aiguille selon la revendication 9, comprenant cinq composants incluant le boîtier (10B), le dispositif d'actionnement (20B), l'élément élastique (30B), la tige de soupape supérieure (37B) et l'adaptateur (40B), dans lequel le tube de compression inférieur (31B), la colonne (32B), le tube de compression supérieur (33B) et la tige de soupape inférieure (34B) sont formés d'un seul tenant afin de composer l'élément élastique (30B).

11. Connecteur médical étanche sans aiguille selon la revendication 3, dans lequel
le tube de compression inférieur (31 C) comporte
une plaque de recouvrement inférieure (311 C) ; et
un orifice central inférieur (312C) s'étendant à travers la plaque de recouvrement inférieure (311 C) ;
le tube de compression supérieur (33C) comporte
une plaque de recouvrement supérieure (331C) ; et
un orifice central supérieur (332C) s'étendant à travers la plaque de recouvrement supérieure (331C) ; et
la tige de soupape inférieure (34C) s'étend vers le bas depuis une surface inférieure de la colonne (32C), la tige de soupape supérieure (37C) s'étend vers le haut depuis une surface supérieure de la colonne (32C), la colonne (32C) comporte une pluralité d'orifices de passage (321C) définis à travers celle-ci, les orifices de passage (321 C) sont espacés autour de la tige de soupape inférieure (34C) et de la tige de soupape supérieure (37C), une partie supérieure du tube de soupape (12C) du boîtier (10C) porte contre une surface inférieure de la plaque de recouvrement inférieure (311 C) du tube de compression inférieur (31 C) afin de pousser la plaque de recouvrement inférieure (311C) vers la colonne (32C) pour fermer des parties inférieures ouvertes des orifices de passage (321 C) de la colonne (32C) de telle sorte que les orifices de passage (321C) ne communiquent pas avec l'espace (36C), la tige de soupape supérieure (37C) comporte une partie élargie (371C) au niveau de son extrémité supérieure, et la partie élargie (371C) porte contre une surface intérieure de l'orifice central supérieur (332C) du tube de compression supérieur (33C) de telle sorte que l'intérieur du tube de compression supérieur (33C) ne communique pas avec l'intérieur de l'adaptateur (40C).

12. Connecteur médical étanche sans aiguille selon la revendication 11, comprenant six composants incluant le boîtier (10C), le dispositif d'actionnement (20C), le tube de compression inférieur (31C), la colonne (32C), le tube de compression supérieur (33C) et l'adaptateur (40C), dans lequel la tige de soupape inférieure (34C) et la tige de soupape supérieure (37C) sont formées d'un seul tenant sur la colonne (32C).

13. Connecteur médical étanche sans aiguille selon l'une quelconque des revendications 1 à 12, dans lequel des parois des tubes de compression inférieur et supérieur (31, 33, 31A, 33A, 31B, 33B, 31C, 33C) sont ondulées.

14. Connecteur médical étanche sans aiguille selon l'une quelconque des revendications 1 à 12, dans lequel des parois des tubes de compression inférieur et supérieur (310, 330, 310A, 330A, 310B, 330B, 310C, 330C) sont sphériques.
